**Europäisches Patentamt**

**European Patent Office**

**Office européen des brevets**

(11) Veröffentlichungsnummer: **0 326 799**
**A2**

(12) **EUROPÄISCHE PATENTANMELDUNG**

(21) Anmeldenummer: **89100077.0**

(22) Anmeldetag: **04.01.89**

(51) Int. Cl.4: **C07K 1/02**

(30) Priorität: **03.02.88 DE 3803124**

(43) Veröffentlichungstag der Anmeldung:
**09.08.89 Patentblatt 89/32**

(84) Benannte Vertragsstaaten:
**AT BE CH DE ES FR GB IT LI NL**

(71) Anmelder: **Degussa Aktiengesellschaft**
**Weissfrauenstrasse 9**
**D-6000 Frankfurt am Main 1(DE)**

(72) Erfinder: **Kula, Maria-Regina, Prof.**
**Selgenbusch 12**
**D-5162 Niederzier/Hambach(DE)**
Erfinder: **Flösheimer, Andreas, Dr.**
**Kopernikusstrasse 33**
**D-5170 Jülich(DE)**

(54) **Verfahren zur enzymatischen Peptidsynthese.**

(57) Peptide werden hergestellt durch Umsetzung von durch eine $N^\alpha$-Formylgruppe geschützten α-Aminocarbonsäureestern oder Peptidestern als Carboxylkomponente mit α-Aminocarbonsäuren, α-Aminocarbonsäureestern oder α-Aminocarbonsäureamiden als Aminokomponente in Gegenwart einer Serin- oder Thiolprotease.

EP 0 326 799 A2

## Verfahren zur enzymatischen Peptidsynthese

Die Erfindung betrifft ein Verfahren zur enzymatischen Peptidsynthese durch Umsetzung eines $N^{\alpha}$-geschützten $\alpha$-Aminocarbonsäureesters oder eines $N^{\alpha}$-geschützten Peptidesters als Carboxylkomponente mit einer $\alpha$-Aminocarbonsäure, einem $\alpha$-Aminocarbonsäureester oder einem $\alpha$-Aminocarbonsäureamid als Aminokomponente in Gegenwart einer Serin- oder Thiolprotease.

Ein derartiges Verfahren ist bereits aus der US-PS 4 339 534 bekannt. Bei diesem bekannten Verfahren werden jedoch als Schutzgruppen für die Carboxylkomponente die Benzoylgruppe, die Acetylgruppe, eine tertiäre Alkoxycarbonylgruppe, die p-Toluolsulfonylgruppe oder die o-Nitrophenylsulfonylgruppe verwendet. Mit derartigen Schutzgruppen versehene Carboxylkomponenten weisen jedoch in einem wäßrigen Reaktionsmedium häufig eine nur geringe Löslichkeit auf.

Das erfindungsgemäße Verfahren ist nun dadurch gekennzeichnet, daß man eine durch eine $N^{\alpha}$-Formylgruppe geschützte Carboxylkomponente einsetzt.

Aus der veröffentlichten europäischen Patentanmeldung 149 594 ist es zwar bereits bekannt, eine durch eine $N^{\alpha}$-Formylgruppe geschützte $\alpha$-Aminocarbonsäure oder ein durch eine $N^{\alpha}$- Formylgruppe geschütztes Peptid als Carboxylkomponente mit einer $\alpha$-Aminocarbonsäure, einem $\alpha$-Aminocarbonsäurealkylester, einem Peptid oder einem Peptidalkylester als Aminokomponente in Gegenwart einer Protease umzusetzen. Bei diesem bekannten Verfahren handelt es sich jedoch um eine sogenannte thermodynamisch kontrollierte Synthese, bei der es erforderlich ist, durch geeignete Maßnahmen ständig das Reaktionsgleichgewicht in Richtung der Peptidbindungsknüpfung zu verschieben. Dieses bekannte Verfahren ist daher im wesentlichen nur zur Synthese kürzerer hydrophober oder Seitenketten-geschützter Peptide anwendbar.

Da beim erfindungsgemäßen Verfahren als Carboxylkomponente ein $\alpha$-Aminocarbonsäureester oder ein Peptidester eingesetzt wird, handelt es sich dabei jedoch um eine sogenannte kinetisch kontrollierte Synthese. Diese ist in der Anwendbarkeit wesentlich universeller als die thermodynamisch kontrollierte Synthese, weil keinerlei Rücksicht auf die Löslichkeitseigenschaften der zu synthetisierenden Peptide genommen werden muß. Dadurch können auch längere Peptide und Peptide mit ungeschützten hydrophilen Aminosäureseitenketten hergestellt werden. Der Bedarf an Enzym ist relativ gering und die Knüpfung der neuen Peptidbindung erfordert nur eine relativ geringe Reaktionszeit.

Das bevorzugte Reaktionsmedium für durch Proteasen katalysierte Reaktionen ist das wäßrige Medium. Um enzymkatalysierte Reaktionen wirtschaftlich betreiben zu können, sind möglichst hohe Substratkonzentrationen erforderlich. Die $N^{\alpha}$-Formylschutzgruppe bewirkt aufgrund ihrer günstigen solubilisierenden Eigenschaften eine erheblich verbesserte Löslichkeit von Aminosäure-bzw. Peptid-Derivaten im wäßrigen Medium. Für die Anwendung in der thermodynamisch kontrollierten Synthese stellt dies jedoch einen grundlegenden Nachteil dar, da die gesteigerten Löslichkeiten der zu synthetisierenden Produkte zu Ausbeuteverminderung bzw. zur Nichtdurchführbarkeit von Peptidbindungsknüpfungen führen kann.

Das erfindungsgemäße Verfahren beinhaltet daher die Anwendung von durch eine $N^{\alpha}$-Formylschutzgruppe geschützten Aminosäure- bzw. Peptid-Derivaten ausschließlich in der kinetisch kontrollierten enzymkatalysierten Peptidsynthese, da hier das eigentliche Anwendungspotential dieser Schutzgruppe liegt.

Als Carboxylkomponenten dienen beim erfindungsgemäßen Verfahren durch eine $N^{\alpha}$-Formlygruppe geschützte $\alpha$-Aminocarbonsäureester oder Peptidester. Als Alkoholkomponente können grundsätzlich beliebige einwertige Alkohole in diesen Estern enthalten sein. Besonders zweckmäßig ist der Einsatz von Niedrigalkylestern, wie Methyl-, Ethyl-, n-Propyl-oder i-Propylestern, oder von Benzylestern. Beispiele für geeignete $\alpha$-Aminocarbonsäureester sind Tryptophan-n-propylester, Tyrosinmethylester, Tyrosin-n-propylester, Phenylalaninmethylester, Phenylalaninethylester, Glutaminsäuredimethylester, Asparaginsäuredimethylester, Asparaginbenzylester, Cystein-n-propylester, Methionin-n-propylester, Methioninmethylester, Lysin-n-propylester, Arginin-n-propylester, Ornithinmethylester, Histidin-n-propylester, Histidinethylester, Isoleucinmethylester, Leucinmethylester, Valinmethylester, Alanin-n-propylester, Serin-n-propylester oder Serinethylester.

Beispiele für geeignete Peptidester sind Tyrosyl-lysin-n-propylester, Tyrosyl-arginin-n-propylester, Tyrosyl-glycin-n-propylester, Tyrosyl-glycinmethylester, Tyrosyl-alanin-n-propylester, Tryptophanyl-lysin-n-propylester oder Tyrosyl-glutaminsäure-$\alpha$-methylester.

Als Aminokomponenten dienen beim erfindungsgemäßen Verfahren $\alpha$-Aminocarbonsäuren, $\alpha$-Aminocarbonsäureester und $\alpha$-Aminocarbonsäureamide. Beispiele für geeignete $\alpha$-Aminocarbonsäuren sind Alanin, Tyrosin, Phenylalanin, Tryptophan, Lysin, Arginin, Leucin, Isoleucin, Serin, Glycin, Methionin, Cystein, Asparagin oder Glutamin.

Beispiele für geeignete $\alpha$-Aminocarbonsäureester sind Lysin-n-propylester, Alanin-n-propylester, Arginin-n-propylester, Glutaminsäuredimethylester, Serinmethylester, Tyrosinmethylester, Leucin-n-propylester, Cystein-n-propylester, Valinmethylester, Valinethylester, Methionin-n-propylester oder Glycin-n-propylester.

Beispiele für geeignete $\alpha$-Aminocarbonsäureamide sind Methioninamid, Leucinamid, Phenylalaninamid, Alaninamid, Serinamid, Tyrosinamid, Argininamid, Lysinamid, Tryptophanamid, Isoleucinamid, Valinamid oder Cysteinamid.

Als Enzyme werden beim erfindungsgemäßen Verfahren Serin- oder Thiolproteasen eingesetzt. Geeignete Enzyme sind beispielsweise $\alpha$-Chymotrypsin, Trypsin, Carboxypeptidase Y, Elastase, Thrombin, Papain, Bromelain, Carboxypeptidase W, Carboxypeptidase C oder Proteinase Glu-C.

Die durch eine $N^\alpha$-Formylgruppe geschützten Carboxylkomponenten können besonders vorteilhaft so hergestellt werden, daß man die bei der Veresterung anfallenden Hydrochloride der $\alpha$-Aminocarbonsäureester oder Peptidester mit einem Gemisch aus Ameisensäure und Essigsäureanhydrid in Gegenwart von mindestens einem

Äquivalent eines sterisch gehinderten tertiären Amins umsetzt. Das Mischungsverhältnis von Ameisensäure und Essigsäureanhydrid kann dabei im Bereich von 10 : 1 bis 4 : 1 Volumenteilen liegen.

Zweckmäßigerweise wird so vorgegangen, daß man das Gemisch auf einem Eisbad vorlegt, das tertiäre Amin als Lösung in Methylenchlorid hinzugibt und dann das umzusetzende Erster-Hydrochlorid unter Rühren zufügt. Die Einführung der Formylgruppe erfordert nur etwa 15 Minuten. Anschließend wird das Reaktionsgemisch mit eiskaltem Wasser auf das etwa doppelte Volumen verdünnt. Die Aufarbeitung kann bei leicht wasserlöslichen Produkten so erfolgen, daß man alle leichtflüchtigen Bestandteile abzieht und den Rückstand durch Ionenaustauscherchromatographie reinigt. Bei weniger wasserlöslichen Produkten kann man die wäßrige Phase mit Kochsalz sättigen und das Produkt mit Methylenchlorid extrahieren. Nach dem Abziehen des Methylenchlorids erhält man dann ein für die weitere Verarbeitung ausreichend reines Produkt.

Das tertiäre Amin muß bei der Herstellung der durch eine $N^\alpha$-Formylgruppe geschützten Carboxylkomponente in einer Menge von mindestens einem Äquivalent eingesetzt werden. Es kann auch im Überschuß, beispielsweise bis zu 3 Äquivalenten, angewandt werden. Als tertiäres Amin wird vorzugsweise N-Ethyldiisopropylamin verwendet. Andere geeignete tertiäre Amine sind beispielsweise N-Ethylmorpholin oder N-Methylmorpholin.

Das erfindungsgemäße Verfahren wird zweckmäßigerweise bei einem pH-Wert im Bereich zwischen 7,0 und 10,5 und bei einer Temperatur im Bereich zwischen Raumtemperatur und 60 °C durchgeführt. Die Umsetzung erfolgt in einem wäßrigen Medium, das gegebenenfalls organische Cosolventien, wie Methanol, Ethanol oder n-Propanol, enthält. Die Carboxylkomponente kann je nach Löslichkeit in einer Konzentration bis zu etwa 1 M eingesetzt werden. Die Aminokomponente wird zweckmäßigerweise im Überschuß über die Carboxylkomponente eingesetzt. Der anzuwendende Überschuß hängt in erster Linie davon ab, wie leicht die unverbrauchte Aminokomponente zurückgewonnen werden kann. Empfehlenswert ist im allgemeinen die Anwendung eines bis zu 5-fachen Überschusses.

Das erfindungsgemäße Verfahren kann diskontinuierlich oder - mit immobilisiertem Enzym oder unter Verwendung eines Enzym-Membran-Reaktors - kontinuierlich durchgeführt werden.

Das als unmittelbares Produkt der enzymatischen Umsetzung anfallende Peptidderivat kann, je nach seiner Zusammensetzung, in der Weise isoliert werden, daß man es beispielsweise im Falle von Peptiden mit hydrophoben Aminosäureseitenketten in einem geeigneten Lösungsmittel, wie Methylenchlorid, löst oder mit einem solchen Lösungsmittel extrahiert und basische Bestandteile, insbesondere den Überschuß an Aminokomponente, mit einer Säure auswäscht. In anderen Fällen kann die Isolierung in an sich bekannter Weise durch präparative HPLC und/oder Ionenaustauscherchromatographie erfolgen.

Bei der Ionenaustauscherchromatographie verwendet man zweckmäßigerweise lyophilisierbare Puffer, z. B. Ammonium-Formiat-Puffer.

Wurde als Aminokomponente ein c$\alpha$-Aminocarbonsäureester eingesetzt, kann die Verseifung des $N^\alpha$-Formylpeptidesters an der Estergruppe entweder enzymatisch mit Hilfe einer Esterase oder durch Behandlung mit einer schwachen Alkalimetallhydroxidlösung erfolgen.

Wurde als Aminokomponente ein $\alpha$-Aminocarbonsäureamid eingesetzt, kann die Abspaltung der Amidgruppe in der Weise erfolgen, daß man das Produkt in einer 20 bis 50 mM Lösung bei pH 6 bis 8 vorlegt, Carboxypeptidase Y oder eine Amidase zugibt und die Verseifung mittels HPLC verfolgt. Ist das Produkt zu über 95 % verseift, wird die Reaktion durch Zugabe von Essigsäure (50 Volumenprozent des Reaktionsgemisches) abgebrochen. Anschließend wird das Gemisch einrotiert und lyophilisiert. Die weitere Aufarbeitung erfolgt über Anionenaustauscherchromatographie und anschließende Gelchromatographie. Als geeignete Gele kommen hierfür in Frage Sephadex G 10 oder Bio Rad P2.

Die $N^\alpha$-Formylschutzgruppe kann durch Behandlung mit verdünnter, beispielsweise 0,3 bis 0,5 M,

3

Salzsäure bei mäßig erhöhter Temperatur bis zu etwa 60 °C innerhalb von etwa 2 bis 6 Stunden abgespalten werden.

Das erfindungsgemäße Verfahren wird durch die nachfolgenden Beispiele näher erläutert:

Beispiel 1:

14 g (60,4 mMol) L-Tyrosinmethylester-Hydrochlorid (Serva 37429) wurden auf einem Eisbad in einem Gemisch aus 80 ml 98 %iger Ameisensäure und 20 ml Essigsäureanhydrid gelöst. Anschließend wurden 66 mMol N-Ethyl-diisopropylamin in 66 ml Methylenchlorid zugegeben und es wurde 15 Minuten lang gerührt. Nach Zusatz von 80 ml gesättigter Kochsalzlösung wurde das Reaktionsgemisch mit 1 N Salzsäure auf pH 2 eingestellt und viermal mit je 70 ml Methylenchlorid ausgeschüttelt. Die vereinigten Methylenchloridphasen wurden zweimal mit je 50 ml salzsaurer halbgesättigter Kochsalzlösung mit pH 2 ausgeschüttelt, über Natriumsulfat getrocknet und unter vermindertem Druck einrotiert. Das Produkt kristallisierte über Nacht im Kühlschrank aus. Es wurde mittels $^1$H-NMR (CDCl$_3$) als N$^\alpha$-Formyl-L-tyrosinmethylester identifiziert : δ = 8,2 ppm (s,1H,-CHO), δ = 4,0 ppm (s,3H,-O-CH$_3$).
Ausbeute: 9,5 g (42,5 mMol; 70 % der Theorie).
Schmelzpunkt: 110 - 116 °C.
$[\alpha]_5^{25}$ : +20,7° (c = 1; MeOH).

447 mg (2mMol) des so hergestellten N$^\alpha$-Formyl-L-tyrosinmethylesters und 400 mg (2,4 mMol) L-Alanin-n-propylester-Hydrochlorid wurden in einem Gemisch aus 4 ml Methanol und 9 ml Wasser gelöst und der pH wurde mit 1 N NaOH auf 9,5 eingestellt. Dann wurden 2,5 mg α-Chymotrypsin (Merck 2307) in Form von 0,2 ml einer wäßrigen Lösung (0,5 N Na$^+$, 0,01 N Ca$^{++}$) zugegeben. Der pH-Wert wurde mittels eines automatischen Titriersystems (Radiometer, VIT 90, TTA 80) konstant gehalten. Nach 5 Minuten kam es zu einer Ausfällung von Produkt. Nach 14 Minuten wurde die Umsetzung durch Zugabe von 50 ml Methanol abgebrochen. Das Reaktionsgemisch wurde eine Stunde stehengelassen, dann unter vermindertem Druck einrotiert. Der Rückstand wurde in 50 ml Methylenchlorid aufgenommen und je einmal mit 8 ml kochsalzgesättigter Sodalösung (pH 10), 8 ml kochsalzgesättigter salzsaurer Lösung (pH 2,0) und 10 ml kochsalzgesättigter salzsaurer Lösung (pH 0,5) ausgeschüttelt. Der gebildete N$^\alpha$-Formyl-L-tyrosyl-L-alanin-n-propylester kristallisierte aus der Methylenchloridlösung spontan aus.
Ausbeute: 447 mg (1,23 mMol; 66 % der Theorie).
Schmelzpunkt: 174 - 174,5 °C.
$[\alpha]_5^{25}$ : +3,24° (c = 1; MeOH).
Aminosäureanalyse: Ala 1,00; Tyr 1,03.

Der Ester wurde mit einem Gemisch aus 50 % verdünnter Natronlauge (pH 10,0) und 50 % Dioxan während 10 Stunden verseift. Anschließend wurde mit 1 N Salzsäure auf pH 2 eingestellt, das Dioxan wurde unter vermindertem Druck abgezogen, und das gebildete N$^\alpha$-Formyl-L-tyrosyl-L-alanin wurde mit Essigester ausgeschüttelt. Die vereinigten Essigesterphasen wurden unter vermindertem Druck einrotiert.

Zur Abspaltung der Formylgruppe wurde der Rückstand in 0,5 M Salzsäure gelöst und 3 Stunden lang auf 56 °C erwärmt. Anschließend wurde das Reaktionsgemisch abgekühlt und das Produkt durch Lyophilisieren isoliert. Ausbeute: quantitativ.
Aminosäureanalyse: Ala 1,00; Tyr 1,03.
$[\alpha]_5^{25}$ : + 9,9° (c = 0,2; 1 N HCl)
Die HPLC-Daten waren mit denjenigen käuflichen Materials identisch.

Vergleichsversuche:

Zu Vergleichszwecken wurde versucht, N$^\alpha$-Formyl-L-tyrosyl-L-alanin-n-propylester durch Umsetzung von N$_\alpha$-Formyl-L-tyrosin als Carboxylkomponente mit L-Alanin-n-propylester-Hydrochlorid als Aminokomponente herzustellen (thermodynamisch kontrollierte Synthese).

Es wurden jeweils 2 mg α-Chymotrypsin in Form von 1 ml einer wäßrigen Lösung (0,5 N Na$^+$; 0,01 N Ca$^{++}$) mit

| N$^\alpha$-Formyl-L-tyrosin | 165 mg | 104,6 mg | 29,3 mg |
| L-Alanin-n-propylester-Hydrochlorid | 102,3 mg | 65,6 mg | 18,4 mg |

versetzt und der pH wurde mittels eines automatischen Titriersystems auf pH 7,5 eingestellt und konstant gehalten.

In allen drei Fällen konnte auch nach einer Reaktionszeit von mehreren Tagen keine Bildung von $N^\alpha$-Formyl-L-tyrosyl-L-alanin-n-propylester beobachtet werden.

Beispiel 2:

9 ml 98 %ige Ameisensäure, 2 ml Essigsäureanhydrid und 6,4 mMol N-Ethyl-diisopropylamin in 6,4 ml Methylenchlorid wurden auf einem Eisbad vorgelegt. Dann wurden 0,9 g (3,18 mMol) L-Tryptophan-n-propylester-Hydrochlorid zugegeben und es wurde 15 Minuten gerührt. Anschließend wurde das Reaktionsgemisch in 10 ml salzsaure gesättigte Kochsalzlösung (pH 3) eingegossen. Das Gemisch wurde viermal mit je 20 ml Methylenchlorid ausgeschüttelt. Die vereinigten Methylenchloridphasen wurden über Natriumsulfat getrocknet und unter vermindertem Druck einrotiert. Das ölige Produkt war bei der dünnschichtchromatographischen und bei der HPLC-Untersuchung einheitlich. Es wurde mittels UV-Analyse (Maxima bei 280 und 220 nm; Wasser), $^1$H-NMR (CDCl$_3$): $\delta$ = 9,8 ppm (s, 1H, H-N$^{in}$), $\delta$ = 8,1 ppm (s, 1 H, N$^\alpha$-CO-H), $\delta$ = 6,8 ppm (d, 1H, N$^\alpha$-H) und Elementaranalyse:

|  | % C | % H | % N | % O |
|---|---|---|---|---|
| Gefunden: | 65,76 | 7,61 | 9,43 | 17,20 |
| Berechnet: | 65,68 | 6,61 | 10,21 | 17,50 |

als $N^\alpha$-Formyl-L-tryptophan-n-propylester identifiziert.
Ausbeute: 650 mg (2,34 mMol; 74 % der Theorie).
$[\alpha]_D^{25}$: +4,9° (c = 2; MeOH).

43 mg (0,16 mMol) des so hergestellten $N^\alpha$-Formyl-L-tryptophan-n-propylesters und 88 mg (0,34 mMol) L-Lysin-n-propylester-Dihydrochlorid wurden in einem Gemisch aus 0,4 ml n-Propanol und 0,5 ml Wasser gelöst und der pH wurde mit 1 N NaOH auf pH 9,2 eingestellt. Dann wurden 1,48 mg $\alpha$-Chymotrypsin in Form von 0,2 ml einer wäßrigen Lösung (0,5 N Na$^+$, 0,01 N Ca$^{++}$) zugegeben. Der pH wurde mittels eines automatischen Titriersystems konstant gehalten. Nach 8 Minuten wurde die Umsetzung durch Zugabe von 0,4 ml Eisessig abgebrochen. Nach 30 Minuten wurde das Reaktionsgemisch unter reduziertem Druck einrotiert und mittels Kationenaustauscherchromatographie (FPLC-Equipment(Pharmacia), CM-Sepharose FF/ 40 mM Natriumacetat/Essigsäure) und anschließende Gelfiltration (Sephadex G 10; 20 %iges wäßriges n-Propanol) aufgearbeitet. Ausbeute: 29,2 mg (0,072 mMol; 45 % der Theorie). Die Identifikation als $N^\alpha$-Formyl-L-Tryptophanyl-L-Lysin-n-propylester erfolgte massenspektroskopisch: M+H$^+$ = 403 (C$_{19}$H$_{30}$N$_4$O$_4$)

Zur Esterverseifung wurde das Produkt in 4,5 ml 50 mM Carbonat-Puffer (pH 9,5) gelöst und mit 2,5 mg Trypsin (Merck 24581; TCPK-treated) versetzt. Nach 4 Minuten wurde das Reaktionsgemisch zwecks Deformylierung mit 3 N HCl auf pH 0,3 eingestellt und während 2 Stunden auf 56 °C erwärmt. Das Reaktionsgemisch wurde anschließend lyophilisiert und das Produkt durch Gelchromatographie (Sephadex G 10; 20 %iges wäßriges n-Propanol; 80 x 2,6 cm Säule; Fluß: 1ml/min) gereinigt und erneut lyophilisiert. Ausbeute: quantitativ.
Aminosäureanalyse (Chymotrypsin-katalysierte Hydrolyse): Trp 1,04; Lys 1,00.

Beispiel 3:

7 g (27 mMol) L-Tyrosin-n-propylester-Hydrochlorid wurden auf einem Eisbad zu einem Gemisch aus 70 ml 98 %iger Ameisensäure und 20 ml Essigsäureanhydrid zugegeben. Dann wurden 28 mMol N-Ethyl-diisopropylamin in 28 ml Methylenchlorid zugesetzt und es wurde 15 Minuten gerührt. Anschließend wurden 60 ml Eiswasser hinzugefügt und das Reaktionsgemisch wurde unter vermindertem Druck einrotiert. Der Rückstand wurde in 50 ml Methylenchlorid aufgenommen und zweimal mit je 20 ml halbgesättigter salzsaurer Kochsalzlösung (pH 3,0) ausgeschüttelt. Die vereinigten Methylenchloridphasen wurden über Natriumsulfat getrocknet und unter vermindertem Druck eingeengt. Der Rückstand wurde in 30 ml Methanol aufgenommen, erneut eingeengt und in weiteren 20 ml Methanol aufgenommen. Nach Zugabe von 80 ml Wasser und Abziehen des Methanols unter vermindertem Druck fiel der gebildete $N^\alpha$-Formyl-L-tyrosin-n-propylester kristallin aus. Er wurde über P$_2$O$_5$ getrocknet. Ausbeute: 4,77 g (19 mMol; 70 % der Theorie).
$[\alpha]_D^{25}$: +23,5° (c = 1; MeOH).

Schmelzpunkt: 113 - 113,5 °C.
$^1$H-NMR: δ = 8,2 ppm (s, 1H, -CHO).
Elemtaranalyse:

|  | %C | %H | %N | %O |
|---|---|---|---|---|
| Gefunden: | 61,45 | 6,78 | 5,46 | 25,5 |
| Berechnet: | 62,14 | 6,82 | 5,57 | 25,5 |

250 mg (1 mMol) des so hergestellten $N^\alpha$-Formyl-L-tyrosin-n-propylesters und 350 mg (1,34 mMol) L-Lysin-n-propylester-Dihydrochlorid wurden in einem Gemisch aus 1,9 ml n-Propanol und 4,8 ml Wasser gelöst und der pH wurde mit 2 N NaOH auf 9,0 eingestellt. Dann wurden 1,9 mg α-Chymotypsin in Form von 0,2 ml einer Wäßrigen Lösung (0,5 N Na$^+$, 0,01 N Ca$^{++}$) zugegeben. Der pH wurde mittels eines automatischen Titriersystems konstant gehalten. Nach 6 Minuten wurde die Umsetzung durch Zugabe von 3 ml Eisessig abgebrochen. Das Reaktionsgemisch wurde analog zu Beispiel 2 aufgearbeitet.
Ausbeute an $N^\alpha$-Formyl-L-tyrosyl-L-lysin-n-propylester (als Acetat): 296 mg (0,67 mMol; 67 % der Theorie).
$[\alpha]_5^{25}$ : +6,4° (c = 1; MeOH).
Schmelzpunkt : > 320 °C
Aminosäureanalyse: Tyr 1,04; Lys 1,00.

Beispiel 4:

29,4 mg (67 μMol) des im Beispiel 3 hergestellten $N^\alpha$-Formyl-L-tyrosyl-L-lysin-n-propylesters und 27,1 mg (147 μMol) L-Methioninamid-Hydrochlorid (Serva 28822) wurden in 0,1 ml n-Propanol und 0,4 ml 0,2 M Carbonatpuffer (pH 9,5) vorgelegt. Der pH wurde mit 2 N NaOH auf 9,2 eingestellt. Dann wurden 0,41 mg Trypsin (Merck 24581; TPCK treated) in Form von 0,2 ml einer wäßrigen Lösung (0,5 N Na$^+$, 0,01 N Ca$^{++}$) zugefügt. Nach einer halben Minute wurde die Umsetzung durch Zugabe von 0,3 ml Essigsäure abgebrochen. Das Reaktionsgemisch wurde analog zu Beispiel 2 aufgearbeitet.
Ausbeute an $N^\alpha$- Formyl-L-tyrosyl-L-lysyl-L-methioninamid:
18,5 mg (39,6 μMol; 59 % der Theorie).
$[\alpha]_5^{25}$ : +3,6° (c = 0,2; 85 % MeOH).
Aminosäureanalyse: Tyr 1,14; Lys 0,98; Met 1,00.

Beispiel 5:

37,7 mg (15 Mol) des im Beispiel 3 hergestellten $N^\alpha$-Formyl-L-tyrosin-n-propylesters und 75 mg (26 μMol) L-Arginin-n-propylester-Dihydrochlorid wurden in 0,3 ml n-Propanol und 0,45 ml 0,2 M Carbonatpuffer (pH 9,5) vorgelegt. Der pH wurde mit 2 N NaOH auf 9,2 eingestellt. Dann wurden 0,9 mg α-Chymotrypsin in Form von 0,2 ml einer wäßrigen Lösung (0,5 N Na$^+$,0,01 N Ca$^{++}$) zugefügt. Der pH-Wert wurde mittels eines automatischen Titriersystems konstant gehalten. Nach 5 Minuten wurde die Umsetzung durch Zugabe von 0,3 ml Essigsäure abgebrochen. Das Reaktionsgemisch wurde analog zu Beispiel 2 aufgearbeitet.
Ausbeute an $N^\alpha$-Formyl-L-tyrosyl-L-arginin-n-propylester: 48,9 mg (9,6 μMol; 64 % der Theorie).
$[\alpha]_5^{25}$ : -2,69° (c = 0,7; 50 % MeOH).
Aminosäureanalyse: Tyr 1,00; Arg 1,10.

Beispiel 6:

100 g (0,46 Mol) L-Phenylalaninmethylester-Hydrochlorid wurden auf dem Eisbad in eine Mischung aus 400 ml 98 %iger Ameisensäure und 75 ml Essigsäureanhydrid gegeben. Anschließend wurden 0,48 Mol Diisopropylethylamin (in 480 ml Methylenchlorid) zugegeben und 15 Minuten bei Raumtemperatur gerührt. Die Aufarbeitung erfolgte entsprechend Beispiel 2. Der gebildete $N^\alpha$-Formyl-L-phenylalanin-methylester wurde mittels $^1$H-NMR (CDCl$_3$) : δ = 8,1 ppm (s, 1 H, H-CO-N$^\alpha$) charakterisiert.
Schmelzpunkt: 43 °C.
$[\alpha]_5^{25}$ : + 24,9° (c = 2; MeOH).
Ausbeute: 70,4 g (0,34 Mol; 74 % der Theorie).

0,83 g (4mMol) des so hergestellten $N^\alpha$-Formyl-L-phenylalaninmethylesters und 1,33 g (8 mMol) L-Leucinamid-Hydrochlorid wurden in 90 ml 50 mM Tris-Puffer (pH 9) gelöst. Der pH wurde mittels 2 N NaOH auf 9,0 eingestellt. Anschließend wurden 35 mg $\alpha$-Chymotrypsin (in 5 ml 0,1 N Na$^*$/0,05 N Ca$^{2*}$-wäßriger Lösung) zugegeben. Nach 15 Minuten wurde die Reaktion durch Zugabe von 350 ml Methanol abgebrochen. Nach weiteren 30 Minuten wurde das Reaktionsgemisch unter vermindertem Druck auf 40 ml eingeengt. Das Produkt kristallisierte in der Kälte aus und wurde aus Methanol/Wasser umkristallisiert.
Schmelzpunkt: > 280 °C.
Das Produkt wurde mittels Massenspektrometrie (M+H$^*$ = 306; $C_{16}H_{23}N_3O_3$) als $N^\alpha$-Formyl-L-phenylalanyl-L-leucinamid identifiziert.
Aminosäureanalyse: Phe 1,00; Leu 1,10.
Ausbeute: 0,76 g (2,5 mMol; 62 % der Theorie).

Beispiel 7:

1,12 g (5 mMol) des in Beispiel 1 hergestellten $N^\alpha$-Formyl-L-tyrosinmethylesters und 8,91 g (0,1 Mol) L-Alanin wurden in 95 ml 100 mM Tris-Puffer (pH 8,6) gelöst. Die Reaktionslösung wurde mit 2 N NaOH auf pH 8,6 eingestellt. Anschließend wurden 20 mg Carboxypeptidase Y (Carbiotech) zugegeben. Während der Reaktion wurde der pH mittels eines Autotitrators konstant gehalten. Nach 30 Minuten wurde die Reaktion durch Zugabe von 50 ml Eisessig abgebrochen. Nach weiteren 30 Minuten wurde das Reaktionsgemisch unter reduziertem Druck auf 25 ml eingeengt. Das Produkt wurde durch zweimalige Gelchromatographie isoliert und gereinigt (5 x 50 cm Säule; Sephadex G 10, wäßrige Lösung; Fluß: 2ml/min). Das gebildete $N^\alpha$-Formyl-L-tyrosyl-L-alanin wurde mittels $^1$H-NMR (CDCl$_3$):
$\delta$ = 8,1 ppm (s, 1 H, H-CO-N$^\alpha$) identifiziert.
Aminosäureanalyse: Tyr 1,00; Ala 0,89.
Ausbeute: 0,56 g (2 mMol; 40 % der Theorie).
Das Produkt wurde anschließend in 500 ml 0,5 N HCl gelöst und bei 56 °C 3 Stunden gerührt. Das deformylierte Dipeptid wies die gleichen HPLC-Laufeigenschaften wie käufliches L-Tyrosyl-L-alanin auf.

## Ansprüche

1. Verfahren zur enzymatischen Peptidsynthese durch Umsetzung eines $N^\alpha$-geschützten $\alpha$-Aminocarbonsäureesters oder eines $N^\alpha$-geschützten Peptidesters als Carboxylkomponente mit einer $\alpha$-Aminocarbonsäure, einem $\alpha$-Aminocarbonsäureester oder einem $\alpha$-Aminocarbonsäureamid als Aminokomponente in Gegenwart einer Serin- oder Thiolprotease, dadurch gekennzeichnet, daß man eine durch eine $N^\alpha$-Formylgruppe geschützte Carboxylkomponente einsetzt.

2. Verfahren nach Anspruch 1, dadurch gekennzeichnet, daß man die durch eine $N^\alpha$-Formylgruppe geschützte Carboxylkomponente durch Umsetzung eines $\alpha$-Aminocarbonsäure- oder Peptidester-Hydrochlorids mit einem Gemisch aus Ameisensäure und Essigsäureanhydrid in Gegenwart von mindestens einem Äquivalent eines sterisch gehinderten tertiären Amins herstellt.

3. Verfahren nach Anspruch 2, dadurch gekennzeichnet, daß man als tertiäres Amin N-Ethyl-diisopropylamin verwendet.